# EUROPEAN PATENT SPECIFICATION

(11) **EP 3 593 882 B1**
(45) Date of publication and mention of the grant of the patent: **16.02.2022**
(21) Application number: 18764285.5
(22) Date of filing: 31.01.2018
(51) Int. Cl.: B01D 29/01, B01D 29/56, B01D 35/30, G01N 1/40, B01L 3/00, G01N 33/02

(54) **FILTER DEVICE AND USE THEREOF IN SOLID SAMPLE PRETREATMENT FOR FOOD SAFETY TESTING**
FILTERVORRICHTUNG UND VERWENDUNG DAVON IN DER VORBEHANDLUNG VON FESTPROBEN FÜR LEBENSMITTELSICHERHEITSPRÜFUNGEN
APPAREIL DE FILTRE ET SON UTILISATION DANS LE PRÉTRAITEMENT D'ÉCHANTILLONS SOLIDES POUR DES TESTS DE SÉCURITÉ ALIMENTAIRE

(30) Priority: 06.03.2017 CN 201710126547
(43) Date of publication of application: 15.01.2020
(73) Proprietor: Shenzhen Bioeasy Biotechnology Co., Ltd, Shenzhen, Guangdong 518101 (CN)
(72) Inventor: YAN, Yiyong, Beijing, 100190 (CN); ZHU, Hai, Beijing, 100190 (CN); CAI, Zhenqing, Beijing, 100190 (CN)
(74) Representative: KATZAROV S.A.
(86) International application number: PCT/CN2018/074794
(87) International publication number: WO 2018/161744

(56) References cited:
- WO-A1-2011/068467
- WO-A1-2011/156258
- WO-A1-2016/149235
- CN-A- 105 158 052
- CN-A- 106 124 713
- CN-A- 106 914 045
- CN-U- 202 057 507
- US-A- 5 208 161
- US-A1- 2003 070 975

## Description

### TECHNICAL FIELD

The present invention relates to the field of food safety testing, and in particular, to a filter device and use of the same in solid sample pretreatment for food safety testing.

### BACKGROUND OF THE INVENTION

In food safety testing, a large number of solid samples need to be treated. Usually, where the substances to be tested need to be extracted from a solid sample, a centrifuge is needed. The liquid to be tested can be obtained by adding the solid sample and the extracting agent into the centrifuge, and rotating the centrifuge at a high speed to separate the solid from the liquid. However, the high-speed centrifuge has large size and heavy weight, and occupies a large lab area, it takes a long time to increase and decrease the rotation speed, and there is also a certain safety risk. Moreover, the high-speed centrifuge is expensive, causing an increase in testing costs. In addition, in rapid food safety testing, the testing of solid samples often cannot be carried out on-site, and has to be transferred to the laboratory, due to limited on-site testing conditions, limited space, non-portable and heavy experimental equipment, and unguaranteed power supply.

US2003/070975A1 discloses a combination filter assembly having a number of filter elements that are arranged in parallel one after the other in the direction of flow, wherein each of the filter elements typically comprises a filter sieve that is stretched across and mounted to a housing element.

WO2011/068467A1 relates to the separation, collection and analysis of debris carried by filters or filtration devices. More particularly, the present disclosure describes various embodiments of systems for separating and collecting debris of different sizes, including a system having a simple, space-efficient design that is capable of processing multiple filter samples simultaneously, and corresponding debris collection and analysis processes.

US5208161A relates to the preparation of samples for assay where filtration and ready access to material retained are required, especially for food samples. US5208161A discloses a filter device comprising a plurality of filter assemblies connected up and down and a collecting component, each of the filter assemblies comprises:
a sample tube, which is in a hollow tubular shape and has an upper end opening and a lower end opening, and
a filter mesh for filtering sample to be filtered when the sample to be filtered is placed on the filter mesh;
each of the filter assemblies further comprises an upper connecting part disposed at the upper end opening of the sample tube and a lower connecting part disposed at the lower end opening of the sample tube, and the lower connecting part of the filter assembly arranged above is connected to the upper connecting part of the filter assembly arranged below;
the collecting component comprises an upper connecting part which is same as the upper connecting part of the filter assembly, and the upper connecting part of the collecting component is connected to the lower connecting part of the filter assembly arranged at the bottom; and the collecting component is connected to the lower end opening of the sample tube of the filter assembly arranged at the bottom for collecting liquid filtered by the filter mesh into a testing container.

Further relevant prior art is disclosed in WO03073945A1 and WO9109660A1.

Thus, there is a need for a filter device that can solve or at least alleviate some or all of the disadvantages of the prior art described above.

### SUMMARY OF THE INVENTION

The technical problem to be solved by the present invention is to provide a filter device, which aims to solve or at least alleviate some or all of the disadvantages of the prior art described above, and the use of the same in solid sample pretreatment for food safety testing.

The invention is set out in the appended set of claims. The technical solutions of the present invention are also described below.

According to an aspect of the present invention, a filter device according to claim 1 is provided.

Preferably, each of the filter assemblies comprises one or more of the filter meshes, and the pore size of the filter mesh arranged above is larger than that of the filter mesh arranged

Preferably, the lower connecting part of the filter assembly arranged above is connected to the lower connecting part of the filter assembly arranged below by means of thread, snap or interference fit.

Preferably, all or part of a plurality of the sample tubes comprised in the plurality of the filter assemblies are configured to be a sample tube with a circular or quadrilateral cross section.

Preferably, the filter mesh is made of glass fiber, nylon, polytetrafluoroethylene, polyvinyl chloride, polypropylene or stainless steel; the sample tube is made of glass, metal materials or polymer materials; and the upper connecting part and the lower connecting part are made of glass, metal materials or polymer materials.

Preferably, the pore size of the filter mesh ranges from 0.2 µm to 1500 µm.

According to another aspect of the present invention, use of the filter device as described above in solid sample pretreatment for food safety testing is provided in claim 7.

According to one or more embodiments of the present invention, the benefit described below can be achieved.

According to the present invention, the disadvantages of the prior art can be alleviated or avoided owing to the filter device.

In some embodiments, solid-liquid separation can be achieved without using a centrifuge in solid sample treatment, and thus costs and time are saved, and the requirements for on-site testing conditions during rapid food safety testing are relaxed.

### BRIEF DESCRIPTION OF THE FIGURES

The features and advantages of the present invention and the technical and industrial significance of the exemplary embodiments will be described with reference to the accompanying drawings, and the same reference numeral in the figures indicates the same element, wherein:
Fig. 1 is a schematic diagram of a filter device according to a first example not part of the present invention;
Fig. 2 is a schematic diagram of a filter device according to a first exemplary embodiment of the present invention; and
Fig. 3 is a schematic perspective view of the filter device according to the first exemplary embodiment of the present invention.

### DETAILED DESCRIPTION OF THE INVENTION

Exemplary embodiments of the present invention will be described in detail below with reference to the accompanying drawings. The description of the exemplary embodiments is for illustrative purpose only, and does not aim to limit the present invention or its use or application. Moreover, the size and scale of the parts in the figures are for illustrative purpose only and do not strictly correspond to the actual products.

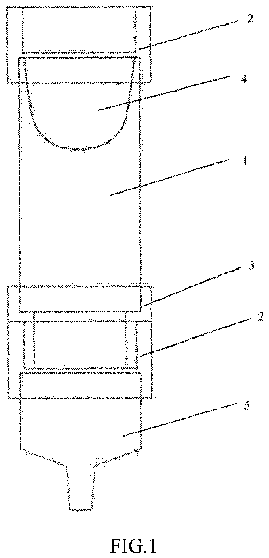

Firstly, a filter device according to the first example not part of the present invention will be described with reference to Fig.1, a schematic diagram of a filter device according to the first example not part of the present invention. As shown in Fig. 1, the filter device according to the first example not part of the present invention comprises a sample tube 1, an upper connecting part 2, a lower connecting part 3, a filter mesh 4, and a collecting component 5, wherein the sample tube 1, the upper connecting part 2, the lower connecting part 3, and the filter mesh 4 constitute a filter assembly of the present invention. When in use, the extracting agent for the liquid to be tested is added into a solid sample to be treated, and after the extraction is completed, the solid sample and the liquid (hereinafter collectively referred to as "sample to be filtered") are poured together onto the filter mesh 4 in the sample tube 1. Under gravity, positive pressure or negative pressure, with the filtration via the filter mesh 4, the solid is retained on the filter mesh 4, and the liquid passes through the filter mesh, then through the collecting component 5, and finally into a testing container, for example a centrifuge tube (not shown), connected to the collecting component 5 for further testing. The collecting component 5 is preferably narrowed down gradually from top to bottom, which facilitates the collection of the liquid.

As shown in Fig. 1, the sample tube 1 is a hollow tubular part, particularly, a sample tube with a circular cross section. Obviously, the sample tube 1 can also be in other shapes, such as a sample tube with a quadrilateral cross section. The sample tube 1 has an upper end opening and a lower end opening. The filter mesh 4 is disposed in the upper end opening for filtering the sample to be filtered when the sample to be filtered is placed on the filter mesh 4. The upper connecting part 2 is also disposed at the upper end opening of the sample tube 1, and the lower connecting part 3 is disposed at the lower end opening of the sample tube 1. Specifically, as shown in Fig. 1, the filter mesh 4 is shaped to have a downwardly-protruding curved-surface, and is sleeved with the upper end opening of the sample tube 1. The filter mesh 4 can be made of materials such as glass fiber, nylon, polytetrafluoroethylene, polyvinyl chloride, polypropylene, or stainless steel.

The mesh size of the filter mesh 4 may be set to range from 0.2 µm to 1500 µm as needed. Moreover, although only one filter mesh 4 is shown in Fig. 1, it will be apparent to those skilled in the art that, a plurality of filter meshes 4 can be disposed as needed. In this case, the plurality of filter meshes 4 may be disposed such that the mesh size of the filter mesh 4 arranged above is larger than that of the filter mesh 4 arranged below, so as to facilitate the sample to be filtered to filtrate through the plurality of filter meshes 4 to achieve multiple filtration, and finally the liquid to be tested which meets the requirements can be obtained. Moreover, in this case, for example, where the plurality of filter meshes 4 are shaped to each have a downwardly-protruding curved surface, there are no special restrictions on the horizontally diameter and vertically downwardly-protruding size of each of the filter meshes 4, as long as they can cooperate with each other to achieve multiple filtration.

In Fig. 1, the upper connecting part 2 and the lower connecting part 3 are parts independent of the sample tube 1, and may be connected to the upper end opening and the lower end opening of the sample tube 1, respectively, by means of thread, snap or interference fit.

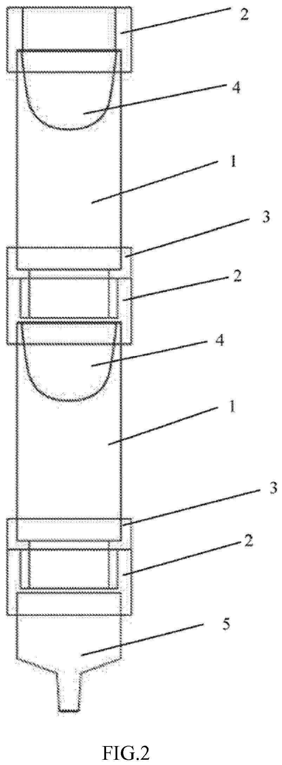

The structure of a filter device according to the first exemplary embodiment of the present invention will be described with reference to Fig.2, a schematic diagram of the filter device according to the first exemplary embodiment of the present invention. As shown in Fig. 2, the filter device according to the first exemplary embodiment of the present invention differs from the filter device according to the first example not part of the present invention in that the former includes two filter assemblies, that is, a first filter assembly arranged above and a second filter assembly arranged below. Each of the two filter assemblies has the same structure as the filter assembly according to the first example not part of the present invention. The lower connecting part 3 of the first filter assembly is connected to the upper connecting part 2 of the second filter assembly by means of thread, snap or interference fit. The collecting component is connected to the lower end opening of the sample tube 1 of the second filter assembly.

Although the filter device according to the first exemplary embodiment as described with reference to Fig. 2 comprises only two filter assemblies, it will be apparent to those skilled in the art that a plurality of filter assemblies, for example, three, four or more filter assemblies, can be disposed, depending on the filtration requirements. Furthermore, in the plurality of filter assemblies, the shape of the plurality of sample tubes 1 can be the same or different.

As a further example not part of the present invention, the filter mesh 4 of the filter assembly arranged at the bottom can be shaped to have a flat surface, and the filter meshes 4 of other filter assemblies arranged above can be shaped to have a downwardly-protruding curved surface. For another example, the filter assembly arranged at the bottom can be provided with one filter mesh 4, while other filter assemblies arranged above can be provided with a plurality of filter meshes 4. This allows for a flexible arrangement of the filter assemblies with different mesh sizes to further meet various requirements of filtration.

Of course, if the filter device does not need gravity to perform filtration, but needs positive pressure or negative pressure to perform filtration, the upper end opening or the lower end opening of the sample tube 1 needs to be connected to a corresponding pressure generating device.

The filter device comprises a plurality of filter meshes, and where a solid sample needs to be treated, an extracting agent is added to the solid sample to be treated, and after the extraction is completed, the sample to be filtered is poured onto the filter meshes 4 arranged at the top of the filter device. Under gravity, positive pressure or negative pressure, with multiple filtration via the plurality of filter meshes 4, the solid is retained on each of the filter meshes 4, and the liquid passes through each of the filter meshes 4, and then through the collecting component 5, and finally into a testing container connected to the collecting component 5 for further testing.

It should be noted that, in the present invention, the positive pressure can be applied in a variety of ways, for example, the positive pressure can be applied downwardly by a pump at the upper end opening of the sample tube 1 arranged at the top of the filter device; the negative pressure can be applied in a variety of ways, for example, the negative pressure can be applied by suction through a silicone tube at the outlet of the collecting component 5 of the filter device.

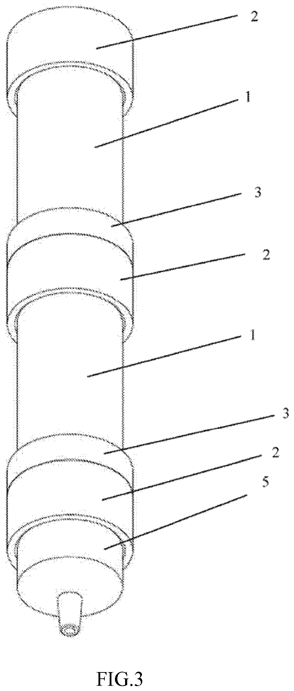

Moreover , in the first example not part of the present invention, where the filter device comprises only one filter assembly, the collecting component 5 further preferably has an upper connecting part 2, and the upper connecting part 2 of the collecting component 5 is connected to the lower connecting part 3 of the filter assembly. In the present invention, where the filter device comprises a plurality of filter assemblies, preferably as shown in Figs. 2-3 , the collecting component also has an upper connecting part 2, and the upper connecting part 2 of the collecting component is connected to the lower connection part 3 of the filter assembly arranged at the bottom. Fig. 3 shows a schematic perspective view of a filter device comprising two filter assemblies.

The embodiments of the present invention have been described in detail above. However, aspects of the present invention are not limited to the embodiments as described above. Various modifications and substitutions can be applied to the embodiments as described above without departing from the scope of the present invention defined by the appended claims.

## Claims

1. A filter device comprising a plurality of filter assemblies connected up and down and a collecting component, wherein each of the filter assemblies comprises:
a sample tube (1), which is in a hollow tubular shape and has an upper end opening and a lower end opening, and
a filter mesh (4), which is disposed in the upper end opening of the sample tube (1) for filtering sample to be filtered when the sample to be filtered is placed on the filter mesh (4); and the filter mesh (4) has a downwardly-protruding curved surface;
each of the filter assemblies further comprises an upper connecting part (2) disposed at the upper end opening of the sample tube (1) and a lower connecting part (3) disposed at the lower end opening of the sample tube (1), and the lower connecting part (3) of the filter assembly arranged above is connected to the upper connecting part (2) of the filter assembly arranged below; and the upper connecting part (2) and the lower connecting part (3) are parts independent of the respective sample tube (1);
the collecting component (5) comprises an upper connecting part (2) which is same as the upper connecting part (2) of the filter assembly, and the upper connecting part (2) of the collecting component (5) is connected to the lower connecting part (3) of the filter assembly arranged at the bottom; and
the collecting component (5) is connected to the lower end opening of the sample tube (1) of the filter assembly arranged at the bottom for collecting liquid filtered by the filter mesh (4) into a testing container.

2. The filter device of claim 1, wherein each of the filter assemblies comprises one or more of the filter meshes (4), and the pore size of the filter mesh (4) arranged above is larger than that of the filter mesh (4) arranged below.

3. The filter device of claim 2, wherein the lower connecting part (3) of the filter assembly arranged above is connected to the upper connecting part (2) of the filter assembly arranged below by means of thread, snap or interference fit.

4. The filter device of claim 2, wherein all or part of a plurality of the sample tubes (1) comprised in the plurality of the filter assemblies are shaped to be a sample tube (1) with a circular or quadrilateral cross section.

5. The filter device of any one of claims 1-4, wherein the filter mesh (4) is made of glass fiber, nylon, polytetrafluoroethylene, polyvinyl chloride, polypropylene or stainless steel; the sample tube (1) is made of glass, metal materials or polymer materials; and the upper connecting part (2) and the lower connecting part (3) are made of glass, metal materials or polymer materials.

6. The filter device of any one of claims 1-4, wherein the pore size of the filter mesh (4) ranges from 0.2 µm to 1500 µm.

7. Use of the filter device of any one of claims 1-6 in solid sample pretreatment for food safety testing.

## Patentansprüche

1. Filtervorrichtung, umfassend eine Mehrzahl von Filterkomponenten, die nach oben und unten verbunden sind, und eine Sammelkomponente, wobei jede der Filterkomponenten umfasst:
ein Probenrohr (1), das hohl tubularförmig ist und das eine obere Endöffnung und eine untere Endöffnung aufweist, und
ein Filtersieb (4), das in der oberen Endöffnung des Probenrohrs (1) angeordnet ist, zum Filtern der zu filtrierenden Probe wenn die zu filtrierende Probe auf dem Filtersieb (4) aufgelegt wird; und das Filtersieb (4) eine nach unten ragende gekrümmte Oberfläche aufweist;
jede der Filterkomponenten ferner ein oberes Verbindungsteil (2), das an der oberen Endöffnung des Probenrohrs (1) angeordnet ist, und ein unteres Verbindungsteil (3), das an der unteren Endöffnung des Probenrohrs (1) angeordnet ist, umfasst, und das untere Verbindungsteil (3) der oben angeordneten Filterkomponente mit dem oberen Verbindungsteil (2) der unten angeordneten Filterkomponente verbunden ist; und das obere Verbindungsteil (2) und das untere Verbindungsteil (3) von dem jeweiligen Probenrohr (1) unabhängige Teile darstellen;
die Sammelkomponente (5) ein oberes Verbindungsteil (2) umfasst, das dem oberen Verbindungsteil (2) der Filterkomponente gleich ist, und das obere Verbindungsteil (2) der Sammelkomponente (5) mit dem unteren Verbindungsteil (3) der am Boden angeordneten Filterkomponente verbunden ist; und
die Sammelkomponente (5) mit der unteren Endöffnung des Probenrohrs (1) der am Boden angeordneten Filterkomponente zum Sammeln von durch das Filtersieb (4) gefilterter Flüssigkeit in einen Testbehälter verbunden ist.

2. Filtervorrichtung nach Anspruch 1, wobei jede der Filterkomponenten eines oder mehrere der Filtersiebe (4) umfasst und die Porengröße des oben angeordneten Filtersiebs (4) größer ist als die des unten angeordneten Filtersiebs (4).

3. Filtervorrichtung nach Anspruch 2, wobei das untere Verbindungsteil (3) der oben angeordneten Filterkomponente mit dem oberen Verbindungsteil (2) der unten angeordneten Filterkomponente mittels Gewinde-, Schnapp- oder Presspassung verbunden ist.

4. Filtervorrichtung nach Anspruch 2, wobei alle oder ein Teil einer Mehrzahl der Probenrohre (1), die in der Mehrzahl der Filterkomponenten enthalten sind, so geformt sind, dass sie ein Probenrohr (1) mit einem kreisförmigen oder viereckigen Querschnitt darstellen.

5. Filtervorrichtung nach einem der Ansprüche 1-4, wobei das Filtersieb (4) aus Glasfaser, Nylon, Polytetrafluorethylen, Polyvinylchlorid, Polypropylen oder Edelstahl hergestellt ist; das Probenrohr (1) aus Glas, Metallmaterialien oder Polymermaterialien hergestellt ist; und das obere Verbindungsteil (2) und das untere Verbindungsteil (3) aus Glas, Metallmaterialien oder Polymermaterialien hergestellt sind.

6. Filtervorrichtung nach einem der Ansprüche 1-4, wobei die Porengröße des Filtersiebs (4) im Bereich von 0,2 µm bis 1500 µm liegt.

7. Verwendung der Filtervorrichtung nach einem der Ansprüche 1-6 bei der Festprobenvorbehandlung zum Testen von Lebensmittelsicherheit.

## Revendications

1. Dispositif de filtrage comprenant une pluralité d'ensembles de filtre connectés verticalement et un collecteur, dans lequel chacun des ensembles de filtre comprend:
un tube d'échantillon (1) en forme tubulaire creuse ayant une ouverture d'extrémité supérieure et une ouverture d'extrémité inférieure, et
un tamis filtrant (4) disposé dans l'ouverture d'extrémité supérieure du tube d'échantillon (1) pour filtrer l'échantillon à filtrer lorsque l'échantillon à filtrer est placé sur le tamis filtrant (4); le tamis filtrant (4) ayant une surface incurvée faisant saillie vers le bas;
chacun des ensembles de filtre comprend en outre une partie de connexion supérieure (2) disposée au niveau de l'ouverture d'extrémité supérieure du tube d'échantillon (1) et une partie de connexion inférieure (3) disposée au niveau de l'ouverture d'extrémité inférieure du tube d'échantillon (1), la partie de connexion inférieure (3) de l'ensemble de filtre disposé au-dessus étant connectée à la partie de connexion supérieure (2) de l'ensemble de filtre disposé en dessous; la partie de connexion supérieure (2) et la partie de connexion inférieure (3) étant des parties indépendantes du tube d'échantillon (1) respectif;
le collecteur (5) comprend une partie de connexion supérieure (2) qui est identique à la partie de connexion supérieure (2) de l'ensemble de filtre, la partie de connexion supérieure (2) du collecteur (5) étant connectée à la partie de connexion inférieure (3) de l'ensemble de filtre disposé au fond; et
le collecteur (5) est connecté à l'ouverture d'extrémité inférieure du tube d'échantillon (1) de l'ensemble de filtre disposé au fond pour collecter liquide filtré par le tamis filtrant (4) dans un récipient de test.

2. Dispositif de filtrage selon la revendication 1, dans lequel chacun des ensembles de filtre comprend un ou plusieurs des tamis filtrant (4), et la taille des pores du tamis filtrant (4) disposé au-dessus est supérieure à celle du tamis filtrant (4) disposé en dessous.

3. Dispositif de filtrage selon la revendication 2, dans lequel la partie de connexion inférieure (3) de l'ensemble de filtre disposé au-dessus est connectée à la partie de connexion supérieure (2) de l'ensemble de filtre disposé en dessous au moyen d'un filetage, d'un encliquetage ou d'un ajustement serré.

4. Dispositif de filtrage selon la revendication 2, dans lequel tout ou partie d'une pluralité de tubes d'échantillon (1) compris dans la pluralité d'ensembles de filtre est formé pour être un tube d'échantillon (1) de section transversale circulaire ou quadrilatérale.

5. Dispositif de filtrage selon l'une quelconque des revendications 1 à 4, dans lequel le tamis filtrant (4) est en fibre de verre, nylon, polytétrafluoroéthylène, chlorure de polyvinyle, polypropylène ou en acier inoxydable; le tube d'échantillon (1) est en verre, matériaux métalliques ou matériaux polymères; et la partie de connexion supérieure (2) et la partie de connexion inférieure (3) sont en verre, matériaux métalliques ou matériaux polymères.

6. Dispositif de filtrage selon l'une quelconque des revendications 1 à 4, dans lequel la taille des pores de la tamis filtrant (4) est comprise entre 0.2 µm et 1500 µm.

7. Utilisation du dispositif de filtrage selon l'une quelconque des revendications 1 à 6 pour prétraitement d'échantillons solides pour des tests de sécurité alimentaire.
